# EUROPÄISCHE PATENTANMELDUNG

(11) **EP 3 020 454 A1**
(43) Veröffentlichungstag der Anmeldung: **18.05.2016**
(21) Anmeldenummer: 14193395.2
(22) Anmeldetag: 17.11.2014
(51) Int. Cl.: A61Q 5/00, A61K 8/87, A61K 8/04

(54) **Polyurethanharnstoffe für Haarstyling-Zusammensetzungen**

(71) Anmelder: Covestro Deutschland AG, 51373 Leverkusen (DE)
(72) Erfinder: VIALA, Sophie, 50935 Köln (DE); DÖRR, Sebastian, 40593 Düsseldorf (DE); NAZARAN, Panthea, 51061 Köln (DE)
(74) Vertreter: Levpat

(57) **Zusammenfassung**

Die Erfindung betrifft eine Haarstyling-Zusammensetzung enthaltend wenigstens ein Treibgas und/oder einen Verdicker und eine wässrige Dispersion eines kationisch hydrophilierten Polyurethanharnstoffs, dadurch gekennzeichnet, dass der Polyurethanharnstoff aufgebaut ist aus
a) wenigstens einem aliphatischen, araliphatischen und/oder cycloaliphatischen Polyisocyanat,
b) Polyolen mit einem zahlenmittleren Molekulargewicht Mn ≥ 400 und ≤ 6000 g/mol und einer Hydroxylfunktionalität von ≥ 1,5 und ≤ 4, umfassend wenigstens ein Polyesterpolyol b1), das eine amorphe Struktur und eine Glasübergangstemperatur Tg > 10 °C aufweist,
c) wenigstens einer kationisch hydrophilierenden Komponente, die mindestens eine gegenüber Isocyanatgruppen reaktive Gruppe und mindestens eine kationische oder potentiell kationische Gruppe aufweist,
d) wenigstens einer aliphatischen, aminofunktionellen Verbindung, die mindestens zwei isocyanatreaktive Aminogruppen aufweist,
e) gegebenenfalls wenigstens einem Alkohol, der mindestens zwei Hydroxylgruppen und eine Molmasse ≥ 60 und ≤ 399 g/mol aufweist und
f) gegebenenfalls wenigstens einer Verbindung die eine gegenüber Isocyanatgruppen reaktive Gruppe aufweist,
sowie die Verwendung des speziellen kationisch hydrophilierten Polyurethanharnstoffs in Haarstyling-Zusammensetzungen. Weiterhin umfasst die Erfindung die Verwendung der erfindungsgemäßen Haarstyling-Zusammensetzung zur Formgebung, Festigung und/oder Fixierung der Haare, sowie ein Verfahren zur Formgebung, Festigung oder Fixierung der Haare unter Verwendung der erfindungsgemäßen Haarstyling-Zusammensetzungen.

## Beschreibung

Die vorliegende Erfindung betrifft Haarstyling-Zusammensetzung enthaltend wenigstens ein Treibgas und/oder einen Verdicker und eine spezielle wässrige Dispersion eines kationisch hydrophilierten Polyurethanharnstoffs, sowie die Verwendung des speziellen kationisch hydrophilierten Polyurethanharnstoffs in Haarstyling-Zusammensetzungen. Weiterhin umfasst die Erfindung die Verwendung der erfindungsgemäßen Haarstyling-Zusammensetzung zur Formgebung, Festigung und/oder Fixierung der Haare, sowie ein Verfahren zur Formgebung, Festigung oder Fixierung der Haare unter Verwendung der erfindungsgemäßen Haarstyling-Zusammensetzungen.

Zur Gestaltung und Stabilisierung vielseitiger Frisuren werden Produkte eingesetzt, die als Haarfestiger oder Haarstyling-Produkte bekannt sind. Haarfestiger gibt es meistens in Form von Schaumfestigern oder Haarsprays, wobei sie sich in ihrer Zusammensetzung kaum unterscheiden. Schaumfestiger werden auf das feuchte Haar als Hilfsmittel zur Modellierung der Frisur aufgetragen. Im Gegensatz hierzu, werden Haarsprays auf trockene fertig gestylte Haare zur Fixierung der Frisur aufgebracht. Neben Haarsprays und Schaumfestiger werden auch Haarfestigergele und Haarfestigerlotionen- oder -cremes angeboten, die zum Styling von nassen oder trockenen Haaren verwendet werden können.

In dem Falle von Haarsprays und Schaumfestigern liegen die Mittel zur Fixierung oder Gestaltung der Frisur üblicherweise als aus Aerosolbehältern, Quetschflaschen oder durch eine Pump-, Sprühoder Schäumvorrichtungen versprühbare Präparate vor, die aus einer alkoholischen oder wäßrigalkoholischen Lösung von filmbildenden natürlichen oder synthetischen Polymeren bestehen. Im Falle von Haarfestigergelen werden die oben beschriebenen Präparate mit herkömmlichen Verdickern auf eine akzeptable Viskosität eingestellt. Haarfestigerlotionen oder -cremes bestehen aus Emulsionen enthaltend filmbildende natürliche oder synthetische Polymere. Diese Polymere können aus der Gruppe der nichtionischen, kationischen, amphoteren oder anionischen Polymeren ausgewählt werden.

Als filmbildende Polymere werden im Stand der Technik häufig anionische oder amphotere Polymere auf Basis von Acrylaten eingesetzt. Bekannt ist aber auch die Verwendung von Polyurethanen und Polyurethanharnstoffen als Filmbildner. So sind beispielsweise in der WO97/17386 A1, WO94/13724 A1, US2005/0169873 A1 und US2008/0025933 A1 Polyurethane, die kationische oder anionische Gruppen aufweisen beschrieben, die für den Einsatz in kosmetischen Produkten, speziell auch für Haarfestiger-Zusammensetzungen geeignet sind.

Die bekannten, auf Polyurethanfilmbildnern basierenden Haarfestiger weisen zwar die üblichen Styling-Eigenschaften, wie einen sehr guten und lang anhaltenden Halt, jedoch nur mangelhafte pflegende Eigenschaften auf. Das Haar lässt sich bei Verwendung dieser Haarstyling-Produkte im nassen Zustand schlecht kämmen und fühlt sich wenig gepflegt an.

Aufgabe der vorliegenden Erfindung war es daher, filmbildende Polymere bereitzustellen, die gleichzeitig einen guten Halt, aber auch pflegende Eigenschaften wie eine gute Kämmbarkeit im nassen Zustand und ein gepflegtes Aussehen und Gefühl der Haare bewirken.

Diese Aufgabe wurde erfindungsgemäß gelöst durch eine Haarstyling-Zusammensetzung enthaltend wenigstens ein Treibgas und/oder einen Verdicker und eine wässrige Dispersion eines kationisch hydrophilierten Polyurethanharnstoffs, dadurch gekennzeichnet, dass der Polyurethanharnstoff aufgebaut ist aus
a) wenigstens einem aliphatischen, araliphatischen und/oder cycloaliphatischen Polyisocyanat,
b) Polyolen mit einem zahlenmittleren Molekulargewicht Mn ≥ 400 und ≤ 6000 g/mol und einer Hydroxylfunktionalität von ≥ 1,5 und ≤ 4, umfassend wenigstens ein Polyesterpolyol b1), das eine amorphe Struktur und eine Glasübergangstemperatur Tg > 10 °C, bestimmt mittels dynamischer Differenzkalorimetrie DSC in Anlehnung an die DIN EN 61006, Verfahren A, aufweist,
c) wenigstens einer kationisch hydrophilierenden Komponente, die mindestens eine gegenüber Isocyanatgruppen reaktive Gruppe und mindestens eine kationische oder potentiell kationische Gruppe aufweist,
d) wenigstens einer aliphatischen, aminofunktionellen Verbindung, die mindestens zwei isocyanatreaktive Aminogruppen aufweist,
e) gegebenenfalls wenigstens einem Alkohol, der mindestens zwei Hydroxylgruppen und eine Molmasse ≥ 60 und ≤ 399 g/mol aufweist und
f) gegebenenfalls wenigstens einer Verbindung die eine gegenüber Isocyanatgruppen reaktive Gruppe aufweist.

Die erfindungsgemäßen Hairstyling-Zusammensetzungen bewirken bei ihrer Anwendung überraschenderweise gleichzeitig einen guten Halt, aber auch pflegende Eigenschaften wie eine gute Kämmbarkeit im nassen Zustand und ein gepflegtes Aussehen und Gefühl der Haare.

Ein weiterer Gegenstand der Erfindung ist Verwendung von wässrigen Dispersion eines kationisch hydrophilierten Polyurethanharnstoffs, der aufgebaut ist aus
a) wenigstens einem aliphatischen, araliphatischen und/oder cycloaliphatischen Polyisocyanat,
b) Polyolen mit einem zahlenmittleren Molekulargewicht Mn ≥ 400 und ≤ 6000 g/mol und einer Hydroxylfunktionalität von ≥ 1,5 und ≤ 4, umfassend wenigstens ein Polyesterpolyol b1), das eine amorphe Struktur und eine Glasübergangstemperatur Tg > 10 °C, bestimmt mittels dynamischer Differenzkalorimetrie DSC in Anlehnung an die DIN EN 61006, Verfahren A, aufweist,
c) wenigstens einer kationisch hydrophilierenden Komponente, die mindestens eine gegenüber Isocyanatgruppen reaktive Gruppe und mindestens eine kationische oder potentiell kationische Gruppe aufweist,
d) wenigstens einer aliphatischen, aminofunktionellen Verbindung, die mindestens zwei isocyanatreaktive Aminogruppen aufweist,
e) gegebenenfalls wenigstens einem Alkohol, der mindestens zwei Hydroxylgruppen und eine Molmasse ≥ 60 und ≤ 399 g/mol aufweist und
f) gegebenenfalls wenigstens einer Verbindung die eine gegenüber Isocyanatgruppen reaktive Gruppe aufweist
in Haarstyling-Zusammensetzungen.

Erfindungsgemäß wird unter einem kationisch hydrophilierten Polyurethanharnstoff ein Polyurethanharnstoff verstanden, der an das Polymergerüst gebundene, kationische oder potentiell kationische Gruppen aufweist. Unter potentiell kationischen gruppen sind solche Gruppen zu verstehen, die durch chemische Reaktion, insbesondere durch Neutralisation in eine kationische Gruppe überführt werden können.

Bevorzugt weisen die in der erfindungsgemäßen Haarstyling-Zusammensetzung enthaltenen Polyurethanharnstoffe einen Gehalt an kationischen und/oder potentiell kationischen Gruppen von ≥ 0,2 und ≤ 5 Milliequivalenten pro g Polymer, besonders bevorzugt von ≥ 0,5 und ≤ 2 Milliequivalenten pro g Polymer und ganz besonders bevorzugt von ≥ 0,6 und ≤ 1 Milliequivalenten pro g Polymer auf.

Polyurethanharnstoffe im Sinne der Erfindung sind polymere Verbindungen, die mindestens zwei bevorzugt mindestens drei Urethan-Gruppen-haltige Wiederholungseinheiten 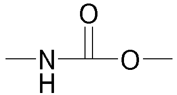 und zudem auch Harnstoff-Gruppen-haltige Wiederholungseinheiten aufweisen:

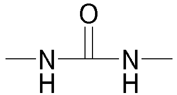

Die Harnstoffgruppen werden dabei bevorzugt durch die Reaktion von ioscyanatfunktionellen Polyurethanpräpolymeren mit Aminogruppen aufweisenden Verbindungen gebildet

Der Polyurethanharnstoff kann neben den Komponenten a) bis f) auch weitere Aufbaukomponenten enthalten, bevorzugt ist er jedoch ausschließlich aus den Komponenten a) bis f) und besonders bevorzugt ausschließlich aus den Komponenten a) bis e) aufgebaut.

Gegenüber Isocyanatgruppen reaktive Gruppen sind im Sinne dieser Erfindung insbesondere primäre und sekundäre Aminogruppen, Hydroxylgruppen und/oder Thiolgruppen.

Das zahlenmittlere Molekulargewicht wird im Rahmen dieser Anmeldung stets bestimmt durch Gelpermeationschromatographie (GPC) in Tetrahydrofuran bei 23°C. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2×PSS SDV linear M, 8×300 mm, 5 µm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Die Komponente a) umfasst wenigstens ein aliphatisches, araliphatisches und/oder cycloaliphatisches Polyisocyanat.

Als Komponente a) geeignete Verbindungen sind beispielsweise 1,4-Butylendiisocyanat, 1,5-Pentamethylendiisocyanat (PDI), 1,6-Hexamethy-lendiisocyanat (HDI), Isophorondiisocyanat (IPDI), 2,2,4- und/oder 2,4,4-Trimethyl-hexa¬methy¬len¬¬diiso¬cyanat, die isomeren Bis-(4,4'-isocyanatocyclo¬hexyl)¬methane oder deren Mischungen beliebigen Isomeren¬gehalts (H12-MDI), 1,4-Cyclohexylendi-isocyanat, 4-Isocyanatomethyl-1,8-octandiisocyanat (Nonantriisocyanat), 1,3- und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysin¬diisocyanate) mit C1-C8-Alkylgruppen, sowie deren Gemische.
Neben den vorstehend genannten Polyisocyanaten können anteilig auch modifizierte Diisocyanate oder Triisocyanate mit Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit einer mittleren NCO-Funktionalität von ≥ 2 und ≤ 4, bevorzugt > 2 bis 2,6 und besonders bevorzugt ≥ 2 und ≤ 2,4.

Bevorzugt werden als Komponente a) HDI, H12-MDI und/oder IPDI eingesetzt.
Besonders bevorzugt umfasst die Komponente a) ≥ 90 Gew.-%, weiterhin bevorzugt ≥ 95 Gew.-% und insbesondere bevorzugt 100 Gew.-% IPDI.
Der Anteil der Komponente a) am Polyurethanharnstoff ist bevorzugt ≥ 5 und ≤ 75 Gew.-%, besonders bevorzugt ≥ 10 und ≤ 65 Gew.-% und ganz besonders bevorzugt > 20 und ≤ 55 Gew.-%, bezogen auf das Gesamtgewicht des Polyurethanharnstoffs. Die Komponenten a) bis f) ergänzen sich dabei zu 100 Gew.-%.

Als Komponente b) werden Polyolen mit einem zahlenmittleren Molekulargewicht Mn ≥ 400 und ≤ 6000 g/mol und einer Hydroxylfunktionalität von ≥ 1,5 und ≤ 4, umfassend wenigstens ein Polyesterpolyol b1), das eine amorphe Struktur und eine Glasübergangstemperatur Tg > 10 °C, bestimmt mittels dynamischer Differenzkalorimetrie DSC in Anlehnung an die DIN EN 61006, Verfahren A, aufweist, eingesetzt.

Bevorzugt weist die Komponente b) ein zahlenmittleres Molekulargewichte von ≥ 600 und ≤ 4000 g/mol, besonders bevorzugt von ≥ 800 und ≤ 3000 g/mol und / oder eine mittlere OH-Funktionalitäten von ≥ 1,8 und ≤ 3 und besonders bevorzugt von ≥ 1,9 und ≤ 2,1 auf.

Als Komponente b) können neben den Polyesterpolyolen b1) insbesondere Polyesterpolyole, Polyetherpolyole, Polycarbonatpolyole, Polyetherpolycarbonatpolyole und/oder Polyesterpolycarbonatpolyole eingesetzt werden. Bevorzugt werden als Komponente b) ausschließlich Polyesterpolye eingesetzt.

Polyesterpolyole sind beispielsweise die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele hierfür geeigneter Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols größer als 2 ist, können zusätzlich auch Monocarbonsäuren, wie Benzoesäure und Hexancarbonsäure mit verwendet werden.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Der Anteil der Komponente b) am Polyurethanharnstoff ist bevorzugt ≥ 5 und ≤ 70 Gew.-%, besonders bevorzugt ≥ 10 und ≤ 60 Gew.-% und ganz besonders bevorzugt > 20 und ≤ 50 Gew.-%, bezogen auf das Gesamtgewicht des Polyurethanharnstoffs. Die Komponenten a) bis f) ergänzen sich dabei zu 100 Gew.-%.

Die Komponente b) umfasst wenigstens ein Polyesterpolyol b1), das eine amorphe Struktur und eine Glasübergangstemperatur Tg > 10 °C, bevorzugt > 10 °C und ≤ 80 °C, besonders bevorzugt ≥ 15 °C und ≤ 50 °C aufweist.

Im Rahmen der Erfindung werden die Glasübergangstemperatur Tg, mittels dynamischer Differenzkalorimetrie (DSC) in Anlehnung an die DIN EN 61006, Verfahren A, bestimmt, wobei ein DSC Gerät (Kalorimeter Pyris Diamond DSC von Perkin-Elmer) verwendet wird, das zur Bestimmung von T_{g} mit Indium und Blei kalibriert ist. Es werden 10 mg der zu untersuchenden Substanz in einen verschließbaren Aluminium-Tiegel eingewogen und dieser verschlossen. Es werden drei unmittelbar aufeinander folgende Durchläufe einer Aufheizungen von -100°C bis +150°C, Heizrate 20 K/min, mit anschließender Abkühlung Kühlrate 320 K/min vorgenommen und die dritte Auf-heizkurve zur Bestimmung der Werte verwendet. Als T_{g} wird die Temperatur bei der halben Höhe einer Glasübergangsstufe bestimmt.

Unter einer amorphen Struktur im Sinne dieser Erfindung wird verstanden, dass die Polyesterpolyole ihrer Struktur keine kristallinen Anteile ausbilden, so dass mittels DSC Messungen nur ein oder mehrere Glasübergangspunkte Tg, aber keine Schmelzpunkte oder Schmelzbereiche für die Polyesterpolyole gefunden werden können.

In einer bevorzugten Ausführungsform der Erfindung umfasst die Komponente b) wenigstens ein Polyesterpolyol b1), das als Aufbaukomponente wenigstens eine aromatische Dicarbonsäure und/oder das entsprechende Carbonsäureanhydrid enthält. Geeignete aromatische Dicarbonsäuren oder Carbonsäureanhydride sind dabei insbesondere Phthalsäure, Isophthalsäure und/oder Terephthalsäure, die korrespondierenden Anhydride, sowie Gemische der genannten Verbindungen.

Bevorzugt beträgt der Anteil an aromatischen Dicarbonsäuren und/oder deren Anhydriden im Polyesterpolyol ≥ 35 Gew.-%, besonders bevorzugt > 45 Gew.-% und ganz besonders bevorzugt ≥ 70 Gew.-%, bezogen auf die Gesamtmasse des Polyesterpolyols.
Es können dabei Mischungen aus aliphatischen und aromatischen Dicarbonsäuren und/oder deren Anhydriden als Säurekomponente bei der Herstellung eingesetzt werden, bevorzugt werden jedoch ausschließlich aromatische Dicarbonsäuren und/oder Anhydride und keine aliphatischen Carbonsäuren und/oder Anhydride als Säurekomponente eingesetzt.
Bevorzugt ist das Polyesterpolyol b1) erhältlich ist aus Säure- und Alkoholkomponenten, wobei als Säurekomponente ausschließlich aromatische Dicarbonsäuren eingesetzt werden.

Bevorzugt enthält das Polyesterpolyol b1) als Diolkomponente Ethylenglykol, 1,4-Butandiol und/oder 1,6-Hexandiol, besonders bevorzugt Ethylenglykol.
Bevorzugt ist das Polyesterpolyol b1) aufgebaut ausschließlich aus Ethylenglykol, 1,4-Butandiol und/oder 1,6-Hexandiol und aromatischen Dicarbonsäure und/oder deren Anhydriden, besonders bevorzugt aus Ethylenglykol und Phthalsäure und/oder Phthalsäureanhydrid.

In einer besonders bevorzugten Ausführungsform der Erfindung umfasst die Komponente b) neben dem Polyesterpolyol b1) ein weiteres Polyesterpolyol b2).

Das Polyesterpolyol b2) enthält bevorzugt als Aufbaukomponente wenigstens eine aliphatische Dicarbonsäure und/oder das entsprechende Carbonsäureanhydrid. Als aliphatische Dicarbonsäure wird dabei bevorzugt Adipinsäure , gegebenenfalls in Mischung mit anderen aliphatischen Dicarbonsäuren und/oder Anhydriden eingesetzt.
Bevorzugt beträgt der Anteil an aliphatischen Dicarbonsäuren und/oder deren Anhydriden im Polyesterpolyol b2) ≥ 40 Gew.-%, besonders bevorzugt ≥ 60 Gew.-% und ganz besonders bevorzugt ≥ 70 Gew.-%, bezogen auf die Gesamtmasse des Polyesterpolyols b2).
Es werden bevorzugt ausschließlich aliphatische Dicarbonsäuren und/oder Anhydride und keine aromatischen Dicarbonsäuren und/oder Anhydride als Säurekomponente eingesetzt.
Bevorzugt enthält das Polyesterpolyol b2) als Diolkomponente verzweigte oder innerhalb der Hauptkette mit Heteroatomen substituierte Diole, insbesondere bevorzugt Butylenglykol, Diethylenglykol, Triethylenglykol, Polyalkylenglykole wie Polyethylenglykol, weiterhin 1,2-Propandiol und/oder Neopentylglykol und ganz besonders bevorzugt Diethylenglykol.
Bevorzugt ist das Polyesterpolyol b2) aufgebaut ausschließlich aus verzweigte oder innerhalb der Hauptkette mit Heteroatomen substituierte Diole, insbesondere bevorzugt Butylenglykol, Diethylenglykol, Triethylenglykol, Polyalkylenglykole wie Polyethylenglykol, weiterhin 1,2-Propandiol und/oder Neopentylglykol? und aliphatischen Dicarbonsäuren und/oder deren Anhydriden, besonders bevorzugt aus Diethylenglykol und Adipinsäure.
Bevorzugt weisen die eingesetzten Polyesterpolyole b2) eine amorphe Struktur auf.
Bevorzugt weisen die Polyesterpolyole b2) einen Glasübergangspunkt Tg, bestimmt mittels DSC, von ≤ 0°C, besonders bevorzugt ≤ - 15°C und ganz besonders bevorzugt ≤ - 25°C auf.

Die Polyesterpolyole b1) und b2) liegen bevorzugt in einem Gewichtsverhältnis b1:b2 von 3:1 bis 1:4 und besonders bevorzugt von 2:1 bis 1:3 und insbesondere bevorzugt von 1:1 bis 1:2,5 vor. In einer besonders bevorzugten Ausführungsform liegt das Polyesterpolyol b2) im Überschuss vor.

Ganz besonders bevorzugt besteht die Komponente b) aus dem Polyesterpolyol b1) oder den Polyesterpolyolen b1) und b2), insbesondere bevorzugt aus den Polyesterpolyolen b1) und b2).

Die Komponente c) umfasst wenigstens eine kationisch hydrophilierenden Komponente, die mindestens eine gegenüber Isocyanatgruppen reaktive Gruppe und mindestens eine kationische oder potentiell kationische Gruppe aufweist.
Bevorzugt weist die Komponente c) wenigstens eine tertiäre Aminogruppe und/oder eine Ammoniumgruppe auf.
Als Komponente c) geeignete Verbindungen sind beispielsweise Tris(hydroxyalkyl)amine, N, N'-bis(hydroxyalkyl)alkylamine, N-hydroxyalkyl-dialkylamine, Tris(aminoalkyl)amine, N, N'-bis(aminoalkyl)alkylamine, N-aminoalkyl-dialkylamine, sowie deren Gemische. Bevorzugt weisen die Alkylreste dabei 2 bis 6 Kohlenstoffatome auf.

Bevorzugt werden als Komponente c) N, N'-bis(hydroxyalkyl)alkylamine eingesetzt. Besonders bevorzugt sind diese ausgewählt aus N-Methyldiethanolamin, N-Ethyldiethanolamin, N-Propyldiethanolamin, N-Methyldipropanlamin, N-Ethyldiproopanolamin, N-Propyldipropanolamin und insbesondere bevorzugt ist die Komponente c) N-Methyldiethanolamin.

Die enthaltenen tertiären Aminogruppen können während oder nach der Herstellung des Polyurethanharnstoffs durch die Zugabe von Säuren teilweise oder komplett neutralisiert werden. Als Säuren werden dabei bevorzugt Phosphorsäure, Schwefelsäure, Halogensäuren und/oder organische Säuren wie Milchsäure, Ameisensäure und/oder Essigsäure, besonders bevorzugt organische Säuren und ganz besonders bevorzugt Essigsäure eingesetzt.

Der Anteil der Komponente c) am Polyurethanharnstoff ist bevorzugt ≥ 1 und ≤ 25 Gew.-%, besonders bevorzugt > 2 und ≤ 20 Gew.-% und ganz besonders bevorzugt > 5 und < 15 Gew.-%, bezogen auf das Gesamtgewicht des Polyurethanharnstoffs. Die Komponenten a) bis f) ergänzen sich dabei zu 100 Gew.-%.

Die Komponente d) umfasst wenigstens eine aliphatische, aminofunktionelle Verbindung, die mindestens zwei isocyanatreaktive Aminogruppen aufweist

Die Verbindungen der Komponente d) weisen bevorzugt keine hydrophilierenden Gruppen, insbesondere keine ionischen und/oder potentiell ionischen Gruppen auf.

Als Komponente d) geeignete Verbindungen sind insbesondere primäre und/oder sekundäre di-oder trifunktionale Amine, bevorzugt primäre und/oder sekundäre difunktionale Amine.

Da die Komponente d) zwei oder mehr isocyanatreaktive Aminogruppen aufweist, dient diese bevorzugt als Kettenverlängerer zum Aufbau höherer Molekulargewichte.

Geeignete Di- und Triamine sind beispielsweise 1,2-Ethandiamin, 1,6-Hexamethylendiamin, 1-amino-3,3,5-trimethyl-5-aminomethyl-cyclohexan (Isophorondiamin, IPDA), Piperazin, 1,4-diaminocyclohexan, bis-(4-aminocyclohexyl)-methan und Diethylenetriamine.

Bevorzugt werden als Komponente d) 1,2-Ethanediamin, 1-Amino-3,3,5-trimethyl-5-aminomethyl-cyclohexan (IPDA) und/oder Diethylentriamin eingesetzt.

Besonders bevorzugt umfasst die Komponente d) ≥ 90 Gew.-%, weiterhin bevorzugt ≥ 95 Gew.-% und insbesondere bevorzugt 100 Gew.-% IPDA.

Der Anteil der Komponente d) am Polyurethanharnstoff ist bevorzugt ≥ 0,5 und ≤ 20 Gew.-%, besonders bevorzugt ≥ 1 und ≤ 15 Gew.-% und ganz besonders bevorzugt ≥ 2 und ≤ 12 Gew.-%, bezogen auf das Gesamtgewicht des Polyurethanharnstoffs. Die Komponenten a) bis f) ergänzen sich dabei zu 100 Gew.-%.

Gegebenenfalls wird der Polyurethanharnstoff zudem aufgebaut aus Komponente e), ein oder mehrere Alkohole, die mindestens zwei Hydroxylgruppen und eine Molmasse von ≥ 60 und ≤ 399 g/mol aufweisen. Beispielsweise können die Polyole des genannten Molmassenbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit, sowie deren Gemische eingesetzt werden.

In einer bevorzugten Ausführungsform der Erfindung wird die Komponente e) eingesetzt.

Die Verbindungen der Komponente e) können prinzipiell nichtionisch hydrophilierende Gruppen aufweisen. Sie weisen jedoch bevorzugt keine ionisch oder nichtionisch hydrophilierenden Gruppen auf.
Der Anteil der Komponente e) am Polyurethanharnstoff ist bevorzugt ≥ 0 und ≤ 10 Gew.-%, besonders bevorzugt ≥ 0,5 und ≤ 6 Gew.-% und ganz besonders bevorzugt ≥ 1 und ≤ 4 Gew.-%, bezogen auf das Gesamtgewicht des Polyurethanharnstoffs. Die Komponenten a) bis f) ergänzen sich dabei zu 100 Gew.-%.

Weiterhin kann der Polyurethanharnstoff aufgebaut sein aus Komponente f), einer oder mehreren Verbindungen, die eine gegenüber Isocyanatgruppen reaktive Gruppe aufweisen, insbesondere Verbindungen die eine Amino- oder Hydroxygruppe aufweisen. Geeignete Verbindungen der Komponente f) sind beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, Methanol, Ethanol, iso-Propanol, n-Propanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmonopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Die Verbindungen der Komponente f) können prinzipiell nichtionisch hydrophilierende Gruppen aufweisen. Sie weisen jedoch bevorzugt keine ionisch oder nichtionisch hydrophilierenden Gruppen auf.

Der Anteil der Komponente f) am Polyurethanharnstoff ist bevorzugt ≥ 0 und ≤ 10 Gew.-%, besonders bevorzugt > 0 und ≤ 3 Gew.-% und ganz besonders bevorzugt 0, bezogen auf das Gesamtgewicht des Polyurethanharnstoffs. Die Komponenten a) bis f) ergänzen sich dabei zu 100 Gew.-%.

In einer bevorzugten Ausführungsform der Erfindung ist entweder die Komponente a) IPDI oder die Komponente d) IPDA oder die Komponente a) IPDI und die Komponente d) IPDA.

Weiterhin bevorzugt ist der Polyurethanharnstoff aufgebaut aus ≥ 10 und ≤ 65 Gew.-% der Komponente a), ≥ 5 und ≤ 70 Gew.-% der Komponente b), ≥ 2 und ≤ 20 Gew.-% der Komponente c), ≥ 1 und ≤ 15 Gew.-% der Komponente d), ≥ 0,5 und ≤ 6 Gew.-% der Komponente e) und ≥ 0 und ≤ 10 Gew.-% der Komponente f), jeweils bezogen auf die Gesamtmasse des Polyurethanharnstoffs, jeweils bezogen auf die Gesamtmasse des Polyurethanharnstoffs, wobei sich die Komponenten a) bis f) zu 100 Gew.-% ergänzen.

In einer bevorzugten Ausführungsform der Erfindung ist der erfindungsgemäß verwendete Polyurethanharnstoff aufgebaut aus
a) wenigstens einem aliphatischen, araliphatischen und/oder cycloaliphatischen Polyisocyanat, das ausgewählt ist aus HDI, H12-MDI und/oder IPDI,
b) Polyesterpoylolen mit einem zahlenmittleren Molekulargewicht Mn ≥ 400 und ≤ 6000 g/mol und einer Hydroxylfunktionalität von ≥ 1,5 und ≤ 4, umfassend wenigstens ein Polyesterpolyol b1), das eine amorphe Struktur und eine Glasübergangstemperatur Tg > 10°C aufweist und das Polyesterpolyol b1) erhältlich ist aus Säure- und Alkoholkomponenten, wobei als Säurekomponente ausschließlich aromatische Dicarbonsäuren eingesetzt werden,
c) wenigstens einer kationisch hydrophilierenden Komponente, die mindestens eine gegenüber Isocyanatgruppen reaktive Gruppe und mindestens eine tertiäre Aminogruppe und/oder eine Ammoniumgruppe aufweist,
d) wenigstens einem aliphatischen primären oder sekundären Diamin, das zwei isocyanatreaktive Aminogruppen und keine ionischen und/oder potentiell ionischen Gruppen aufweist,
e) wenigstens einem Alkohol, der mindestens zwei Hydroxylgruppen und eine Molmasse ≥ 60und ≤ 399 g/mol aufweist und
f) gegebenenfalls wenigstens einer Verbindung die eine gegenüber Isocyanatgruppen reaktive Gruppe aufweist.

Weiterhin bevorzugt ist der Polyurethanharnstoff dieser Ausführungsform aufgebaut aus ≥ 10 und ≤ 65 Gew.-% der Komponente a), ≥ 5 und ≤ 70 Gew.-% der Komponente b), ≥ 2 und ≤ 20 Gew.- % der Komponente c), ≥ 1 und ≤ 15 Gew.-% der Komponente d), ≥ 0,5 und ≤ 6 Gew.-% der Komponente e) und ≥ 0 und ≤ 10 Gew.-% der Komponente f), jeweils bezogen auf die Gesamtmasse des Polyurethanharnstoffs, jeweils bezogen auf die Gesamtmasse des Polyurethanharnstoffs, wobei sich die Komponenten a) bis f) zu 100 Gew.-% ergänzen.

In einer besonders bevorzugten Ausführungsform der Erfindung ist der erfindungsgemäß verwendete Polyurethanharnstoff ausschließlich aufgebaut aus
a) wenigstens einem aliphatischen, araliphatischen und/oder cycloaliphatischen Polyisocyanat, wobei die Komponente a) ≥ 95 Gew.-% an IPDI umfasst,
b) Polyesterpolyol b1), das eine amorphe Struktur und eine Glasübergangstemperatur > 10°C und ≤ 80°C aufweist und das Polyesterpolyol b1) erhältlich ist aus Säure- und Alkoholkomponenten, wobei als Säurekomponente ausschließlich aromatische Dicarbonsäuren eingesetzt werden und Polyesterpolyol b2), das als Säurekomponente wenigstens eine aliphatische Dicarbonsäuren oder die korrespondierenden Anhydride enthält und eine amorphe Struktur aufweist,
c) wenigstens einer kationisch hydrophilierenden Komponente, die ausgewählt ist aus N, N'-bis(hydroxyalkyl)alkylaminen,
d) wenigstens einer aliphatischen, aminofunktionellen Verbindung, die mindestens zwei isocyanatreaktive Aminogruppen aufweist, wobei die Komponente a) ≥ 95 Gew.-% an IPDA umfasst,
e) wenigstens einem Alkohol, der mindestens zwei Hydroxylgruppen und eine Molmasse ≥ 60 und ≤ 399 g/mol aufweist,
f) gegebenenfalls wenigstens einer Verbindung die eine gegenüber Isocyanatgruppen reaktive Gruppe aufweist und keine ionisch oder nichtionisch hydrophilierenden Gruppen enthält.

Weiterhin bevorzugt ist der Polyurethanharnstoff der vorstehenden Ausführungsform aufgebaut aus ≥ 10 und ≤ 65 Gew.-% der Komponente a), ≥ 5 und ≤ 70 Gew.-% der Komponente b), ≥ 2 und ≤ 20 -Gew.-% der Komponente c), ≥ 1 und ≤ 15 Gew.-% der Komponente d), ≥ 0,5 und ≤ 6 Gew.-% der Komponente e) und ≥ 0 und ≤ 10 Gew.-% der Komponente f), jeweils bezogen auf die Gesamtmasse des Polyurethanharnstoffs, jeweils bezogen auf die Gesamtmasse des Polyurethanharnstoffs, wobei sich die Komponenten a) bis f) zu 100 Gew.-% ergänzen.

Vorteilhaft weist der Polyurethanharnstoff ein zahlenmittleres Molekulargewicht Mn ≥ 3000 und ≤ 50000 g/mol, besonders vorteilhaft ≥ 5000 und ≤ 30000 g/mol, auf.

Für die Herstellung der Polyurethanharnstoffe werden bevorzugt die Komponenten a), b) und c) sowie gegebenenfalls e) und f) zur Herstellung eines NCO-terminierten Präpolymers ganz oder teilweise vorgelegt, gegebenenfalls mit einem gegenüber Isocyanatgruppen inerten Lösungsmittel verdünnt und auf Temperaturen im Bereich von 50 bis 120°C aufgeheizt. Die Herstellung der Präpolymere erfolgt dabei bevorzugt in einem Schritt, kann aber auch stufenweise erfolgen.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton. Auch die Zugabe anderer Lösemittel ohne isocyanatreaktive Gruppen ist möglich, bevorzugt werden solche Lösemittel eingesetzt, die mit Wasser mischbar sind.

Zur Beschleunigung der Isocyanatadditionsreaktion können die in der Polyurethan-Chemie bekannten Katalysatoren eingesetzt werden. In einer bevorzugten Variante wird jedoch ohne den Zusatz von Urethanisierungs-Katalysatoren gearbeitet.

Bei der Herstellung der NCO-terminierten Präpolymere aus den Komponenten a), b) und c) sowie gegebenenfalls e) und f) beträgt das Stoffmengenverhältnis von Isocyanat-Gruppen zu isocyanatreaktiven Gruppen im allgemeinen > 1,05 und ≤ 2,5, bevorzugt ≥ 1,15 und ≤ 1,95, besonders bevorzugt ≥ 1,2 und ≤ 1,7.

In einem sich anschließenden Schritt wird bevorzugt dann das im ersten Schritt erhaltene NCOterminierte Präpolymer ganz oder teilweise mit der Komponente d) sowie gegebenenfalls den Komponenten c), e) und f) umgesetzt. Bevorzugt wird die Komponente c) nicht eingesetzt, besonders bevorzugt erfolgt die Umsetzung nur mit der Komponente d). Diese Umsetzung wird im allgemeinen als Kettenverlängerung, bzw. im Fall der Komponente f) als Kettenabbruch bezeichnet. Die Umsetzung kann in einem Schritt oder stufenweise erfolgen.

Bevorzugt wird dabei das NCO-terminierte Präpolymer vorgelegt und die Komponenten d) sowie gegebenenfalls c), e) und f) zudosiert. Die Komponenten d) und gegebenenfalls c), e) und f) können dabei auch stufenweise in mehreren Schritten, insbesondere in zwei Schritten, zugegeben werden. Die Komponenten d) sowie gegebenenfalls c), e) und f) können in Wasser oder organischen Lösemitteln eingesetzt werden.

Die Zugabe der Komponenten d) sowie gegebenenfalls c), e) und f) erfolgt bevorzugt bei Temperaturen von 10 bis 100°C, vorzugsweise 25 bis 60 °C.

Der Kettenverlängerungsgrad, also das molare Verhältnis von NCO-reaktiven Gruppen der zur Kettenverlängerung und Kettenabbruch eingesetzten Komponenten d) sowie gegebenenfalls c), e) und f) zu freien NCO-Gruppen des Präpolymers, liegt im allgemeinen > 25 und ≤ 150%, bevorzugt ≥ 50 und ≤ 120%, besonders bevorzugt > 40 und ≤ 100%.

Enthält die Komponente c) potentiell kationische Gruppen, so können diese durch Neutralisation, bevorzugt mit einer der oben genannten Säuren, ganz oder teilweise in kationische Gruppen überführt werden.

Die Stoffmenge der Säure beträgt bevorzugt zwischen 50 und 125 mol%, besonders bevorzugt zwischen 70 und 100 mol%, der Stoffmenge der zu neutralisierenden Gruppen. Die Neutralisation kann vor, während oder nach der Umsetzung des NCO-terminierten Präpolymers erfolgen.

Der Polyurethanharnstoff ist bevorzugt erhältlich durch Umsetzung der Komponenten a), b), c) und gegebenenfalls e) und f) zu einem Isocyanat-terminierten Präpolymer, anschließender Umsetzung des Präpolymers mit der Komponente d) und gegebenenfalls den Komponenten e) und f) und falls die Komponente c) eine potentiell kationische Gruppe umfasst Neutralisation des Polyurethanharnstoffs durch eine Säure vor, während oder nach der Umsetzung des Isocyanat-terminierten Präpolymers mit der Komponente d) und gegebenenfalls den Komponenten e) und f).

Die Dispergierung des Polyurethanharnstoffs in Wasser kann vor, während oder nach der Umsetzung des NCO-terminierten Präpolymers erfolgen. Die Dispergierung kann während oder nach der Neutralisation erfolgen. Die Neutralisation kann auch gleichzeitig mit der Dispergierung erfolgen, in dem das Dispergierwasser bereits das Neutralisationsmittel enthält.

Die Dispergierung erfolgt bevorzugt im Anschluss an die Umsetzung des-NCO terminierten Präpolymers. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Zur Herstellung der Polyurethandispersion können alle aus dem Stand der Technik bekannten Verfahren wie z. B. Prepolymer-Mischverfahren, Acetonverfahren oder Schmelzdispergierverfahren verwendet werden. Bevorzugt wird das Aceton-Verfahren angewandt.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den so hergestellten Polyurethan-Dispersionen beträgt bevorzugt 0 bis 10 Gew.-%, besonders bevorzugt 0 bis 3 Gew.-%, bezogen auf die gesamte Dispersion.

Der Feststoffanteil des Polyurethanharnstoffs an der erfindungsgemäß eingesetzten Polyurethan-harnstoffdispersion liegt dabei bevorzugt ≥ 10 und ≤ 80 Gew.-%, besonders bevorzugt ≥ 15 und ≤ 60 Gew.-% und ganz besonders bevorzugt bei ≥ 20 und ≤ 50 Gew.-%, bezogen auf das Gesamtgewicht der Polyurethanharnstoffdispersion.

Die Polyurethanharnstoffdispersion weist bevorzugt eine Viskosität ≥ 10 und ≤ 10000 mPas, besonders bevorzugt Viskosität > 50 und ≤ 15000 mPas, bestimmt mittels Rotationsviskosimetrie nach DIN 53019 bei 23°C, auf.

Die erfindungsgemäße Haarstyling-Zusammensetzung enthält bevorzugt ≥ 0,1 und ≤ 40 Gew.-% des oben beschriebenen kationischen Polyurethanharnstoffs (bezogen auf den Aktivstoff selbst und nicht die wässrige Dispersion), insbesondere bevorzugt ≥ 0,2 und ≤ 30 Gew.-% und ganz besonders bevorzugt ≥ 1 und ≤ 20Gew.-%, jeweils bezogen auf das Gesamtgewicht der Zusammensetzung. Die erfindungsgemäße Haarstyling-Zusammensetzung enthält bevorzugt ≥ 2 und ≤ 90 Gew.-% der wässrigen Polyurethanharnstoffdispersion, insbesondere bevorzugt ≥ 10 und ≤ 80 Gew.-% und ganz besonders bevorzugt ≥ 20 und ≤ 70 Gew.-%, jeweils bezogen auf das Gesamtgewicht der Zusammensetzung.

Die erfindungsgemäßen Haarstyling-Zusammensetzungen liegen bevorzugt in Form von Gelen, Lotionen, Cremes, Fluiden, Pumpsprays, Aerosol-Schäumen, Schäumenum und Aerosol-sprays vor, besonders bevorzugt in Form von Gelen, Lotionen, Cremes, Fluiden, Pumpsprays, Aerosol-Schäumen oder Schäumen.

Die erfindungsgemäßen Haarstyling-Zusammensetzungen können vorteilhafter Weise in einer Pumpspray- oder Aerosolverpackung vorliegen. Sie können auch vorteilhaft mit einem Treibgas aufgeschäumt werden. Dementsprechend sind Pumpsprays, Aerosolverpacken und Schaumspender, die die erfindungsgemäße Zusammensetzung enthalten, ebenfalls Bestandteil der Erfindung.

Die erfindungsgemäßen Haarstyling-Zusammensetzungen weisen bevorzugte eine Viskosität von ≥ 0,5 und ≤ 20000 mPas auf. Gelförmige Zusammensetzungen weisen besonders bevorzugt eine Viskosität von ≥ 2000 und ≤ 20000 mPas und ganz besonders bevorzugt von ≥ 5000 und ≤ 10000 mPas auf. Sprühbare Zusammensetzungen für Sprays, Fluide und Aerosole weisen besonders bevorzugt eine Viskosität von ≥ 0,5 und ≤ 500 mPas und ganz besonders bevorzugt von ≥ 1 und ≤ 200 mPas auf. Lotionen und Cremes weisen besonders bevorzugt eine Viskosität von ≥ 200 und ≤ 10000 mPas und ganz besonders bevorzugt von ≥ 500 und ≤ 5000 mPas auf.

Die angegebenen Viskositäten werden mittels Rotationsviskosimetrie nach DIN 53019 bei 23°C mit einem Rotationsviskosimeter der Firma Anton Paar Germany GmbH, Ostfildern, DE, bei einer Scherrate von 10 s⁻¹ bestimmt.

In einer bevorzugten Ausführungsform der Erfindung enthält die Haarstyling-Zusammensetzung wenigstens ein Treibgas.
Für die erfindungsgemäße Haarstyling-Zusammensetzung geeignete Treibgase sind Kohlenwasserstoffe wie Propan, Isobutan und n-Butan sowie deren Mischungen. Druckluft, Fluorkohlenwasserstoffe, wie beispielsweise 1,2-Difluoroethan (Treibmittel 152A), Kohlendioxid, Stickstoff, Stickstoffdioxid und Dimethylether sowie Mischungen all dieser Gase können ebenfalls verwendet werden. Bevorzugt werden in der erfindungsgemäßen Haarstyling Zusammensetzung Dimethylether oder Gemische aus Propan und Butan eingesetzt.

Die Treibgase machen bevorzugt ≥ 0,5 und ≤ 80 Gew.-%, besonders bevorzugt ≥ 1 und ≤ 65 Gew.-% und ganz besonders bevorzugt > 5 und ≤ 50 Gew.-%, bezogen auf das das Gesamtgewicht der Haarstyling-Zusammensetzung, aus.

Für die erfindungsgemäßen Haarstyling-Zusammensetzungen vorteilhafte Verdicker sind:
- Vernetzte oder nichtvernetzte Acrylsäure oder Methacrylsäure Homo- oder Copolymere. Hierzu gehören vernetzte Homopolymere von Methacrylsäure oder Acrylsäure, Copolymerisate aus Acrylsäure und / oder Methacrylsäure und Monomeren, die von anderen Acryl- oder Vinylmonomeren abgeleitet sind, wie C10-30 Alkylacrylate, C10-30-Alkylmethacrylate und Vinylacetat.
- Verdickende Polymere natürlicher Herkunft beispielweise auf Cellulosebasis, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure.
- Nichtionische, anionische, kationische oder amphotere assoziative Polymere z.B. auf Basis von Polyethylenglycole und ihre Derivate, oder Polyurethane.
- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methylpropansulfonsäure angegeben werden.

Besondere vorteilhafte Verdicker sind verdickende Polymere natürlicher Herkunft, vernetzte Acrylsäure oder Methacrylsäure Homo- oder Copolymere und vernetzte Copolymere von 2-Acrylamido-2-methylpropansulfonsäure.

Ganz besondere vorteilhafte Verdicker sind Xanthangummi, wie die unter den Bezeichnungen Keltrol^{®} und Kelza^{®} von der Firma CP Kelco angebotenen Produkte oder die Produkte der Firma RHODIA mit der Bezeichnung Rhodopol und Guargummi, wie die unter der Bezeichnung Jaguar^{®} HP105 von der Firma RHODIA erhältlichen Produkte.

Verdicker werden im Allgemeinen in einer Konzentration von ≥ 0 und ≤ 2 Gew.-%, vorzugsweise von ≥ 0,1 und ≤ 1 Gew.-% eingesetzt.

In den erfindungsgemäßen Zusammensetzungen können des weiteren haarpflegende Stoffe enthalten sein. Als Pflegestoffe können bevorzugt Panthenol und/oder cyclische Polydimethylsiloxane (Cyclomethicone) oder Silikontenside (Polyethermodifizierte Siloxane) vom Typ Dimethicone Copolyol oder Simethicon zum Einsatz kommen. Cyclomethicone werden u. a. unter der Handelsbezeichnungen Abil^{®} K4 von der Firma Goldschmidt oder z.B. DC 244, DC 245 oder DC 345 von der Firma Dow Corning angeboten. Dimethicon-Copolyole werden z. B. unter der Handelsbezeichnung DC 193 von der Firma Dow Corning bzw. Belsil^{®} DM 6031 von der Firma Wacker angeboten.

Die erfindungsgemäßen Haarstyling-Zusammensetzungen können neben dem oben beschriebenen Polyurethanharnstoffen auch weitere geeignete Filmbildner enthalten, welche insbesondere auch zur Festigung und zum Styling der Haare beitragen können.

Der Anteil eines oder mehrerer weiterer Filmbildner kann von ≥ 0 und ≤ 20 Gew-% und insbesondere ≥ 0 und ≤ 10 Gew-%, bezogen auf die gesamte Formulierung, betragen.

Vorteilhaft werden der oder die weiteren Filmbildner aus der Gruppe der nichtionischen, anionischen, amphoteren und / oder kationischen Polymere und Mischungen hieraus ausgewählt, bevorzugt sind dabei nichtionische und/oder kationische Polymere.

Geeignete nichtionische Polymere, die in der erfindungsgemäßen Zusammensetzung alleine oder in Mischungen, vorzugsweise auch mit anionischen und / oder amphoteren und / oder zwitterionischen Polymeren enthalten sein können, sind ausgewählt aus der Gruppe:
- Polyalkyloxazoline;
- Vinylacetat Homo- oder Copolymerisate, wobei. hierzu beispielweise Copolymerisate aus Vinylacetat und Acrylsäureester, Copolymerisate aus Vinylacetat und Ethylen, Copolymerisate aus Vinylacetat und Maleinsäureester gehören;
- Acrylsäureester Copolymerisate wie z. B. die Copolymerisate aus Alkyl-Acrylat und Alkyl-Methacrylat, Copolymerisate aus Alkyl-Acrylat und Urethanen;
- Copolymerisate aus Acrylnitril und nichtionischem monomer ausgewählt aus Butadien und (Meth)Acrylat;
- Styrol Homo- und Copolymerisate, wobei hierzu beispielweise Homopolystyrol, Copolymerisate aus Styrol und Alkyl-(Meth)Acrylat , Coplymerisate aus Styrol, AlkylMethacrylat und Alkyl-Acrylat, Copolymerisate aus Styrol und Butadien, Copolymerisate aus Styrol, Butadien und Vinylpyridin gehören;
- Polyamide;
- Vinyllactame Homo- oder Copolymere, wie Vinylpyrrolidon-Homo- oder Copolymerisate; wobei hierzu beispielweise Polyvinylpyrrolidon, Polyvinylcaprolactam, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat gehören;
- Polysiloxane; und
- Homopolymere des N-Vinylformamids.

Besondere bevorzugte nichtionische Polymere sind Acrylsäureester Copolymerisate, Homopolymere des Vinylpyrrolidons und Copolymere sowie Polyvinylcaprolactam.

Ganz besondere bevorzugte nichtionische Polymere sind Homopolymere des Vinylpyrrolidons z. B. Luviskol® K der Firma BASF, Copolymerisate aus Vinylpyrrolidon und Vinylacetat z. B. Luviskol® VA Typen der Firma BASF oder PVPVA® S630L der Firma ISP, Terpolymere aus Vinylpyrrolidon, Vinylacetat und Propionat wie z. B. Luviskol® VAP von BASF und Polyvinylcaprolactame z.B. Luviskol® PLUS der Firma BASF.

Vorteilhafte amphotere Polymere können unter den Polymeren ausgewählt werden, die statistisch in der Polymerkette verteilte Einheiten A und B enthalten, wobei A für eine Einheit steht, die von einem Monomer mit mindestens einem basischen Stickstoffatom abgeleitet ist, und B eine Einheit darstellt, die von einem sauren Monomer stammt, das eine oder mehrere Carboxy- oder Sulfonsäuregruppen aufweist. Alternativ können A und B Gruppen sein, die von zwitterionischen Carboxybetainmonomeren oder Sulfobetainmonomeren abgeleitet sind. A und B können auch eine kationische Polymerkette sein, die primäre, sekundäre, tertiäre oder quartanäre Gruppen enthalten, wobei mindestens eine Aminogruppe eine Carboxygruppe oder Sulfonsäuregruppe trägt, die über eine Kohlenwasserstoffgruppe gebunden ist, oder A und Bsind Teil einer Polymerkette mit Ethylen-α,β-dicarbonsäureeinheit, bei der die Carbonsäuregruppen mit einem Polyamin umgesetzt wurden, das eine oder mehrere primäre oder sekundäre Aminogruppen enthält.

Besondere vorteilhafte amphotore Polymere sind:
- Polymere, die bei der Copolymerisation eines von einer Vinylverbindung mit Carboxygruppe abgeleiteten Monomers, wie insbesondere Acrylsäure, Methacrylsäure, Maleinsäure, α-Chloracrylsäure, und einem basischen Monomer gebildet werden. Das basische Monomer ist von einer Vinylverbindung abgeleitet, die substituiert ist und mindestens ein basisches Atom enthält, wie insbesondere Dialkylaminoalkylmethacrylat und -acrylat, Dialkylaminoalkylmethacrylamid und -acrylamid. Solche Verbindungen sind in dem amerikanischen Patent US 3,836,537 beschrieben worden.
- Polymere mit Einheiten, die von: a) mindestens einem Monomer, das unter den Acrylamiden oder Methacrylamiden ausgewählt ist, die am Stickstoffatom mit einer Alkylgruppe substituiert sind, b) mindestens einem sauren Comonomer, das eine oder mehrere reaktive Carboxygruppen enthält, und c) mindestens einem basischen Comonomer, wie Estern von Acrylsäure und Methacrylsäure mit primären, sekundären, tertiären und quartären Aminosubstituenten und dem Quaternisierungsprodukt von Dimethylaminoethylmethacrylat mit Dimethylsulfat oder Diethylsulfat abgeleitet sind.

Besonders bevorzugte N-substituierte Acrylamide oder Methacrylamide sind Verbindungen, deren Alkylgruppen 2 bis 12 Kohlenstoffatome enthalten. Ganz besonders bevorzugt sind N-Ethylacrylamid, N-t-Butylacrylamid, N-t-Octylacrylamid, N-Octylacrylamid, N-Decylacrylamid, N-Dodecylacrylamid sowie die entsprechenden Methacrylamide.

Geeignete saure Comonomere sind insbesondere aus der Gruppe Acrylsäure, Methacrylsäure, Crotonsäure, Itaconsäure, Maleinsäure, Fumarsäure sowie den Alkylmonoestern mit 1 bis 4 Kohlenstoffatomen von Maleinsäure, Maleinsäureanhydrid, Fumarsäure oder Fumarsäureanhydrid ausgewählt.

Bevorzugte basische Comonomere sind Aminoethylmethacrylat, Butylaminoethylmethacrylat, N,N-Dimethylaminoethylmethacrylat, N-t-Butylaminoethylmethacrylat.
- Vernetzte und ganz oder teilweise acylierte Polyaminoamide, die von Polyaminoamiden der folgenden allgemeinen Formel

   -[CO-R-CO-Z]-

   abgeleitet sind, worin R eine zweiwertige Gruppe ist, die von einer gesättigten Dicarbonsäure, einer aliphatischen Mono- oder Dicarbonsäure mit ethylenischer Doppelbindung, einem Ester dieser Säuren mit einem niederen Alkanol mit 1 bis 6 Kohlenstoffatomen oder einer Gruppe abgeleitet ist, die bei der Addition einer dieser Säuren an ein bisprimäres oder bis-sekundäres Amin entsteht, und Z eine Gruppe ist, die von einem bis-primären, mono-oder bis-sekundären Polyalkylenpolyamin abgeleitet ist.

Die gesättigten Carbonsäuren sind vorzugsweise unter den Säuren mit 6 bis 10 Kohlenstoffatomen, wie Adipinsäure, 2,2,4-Trimethyladipinsäure und 2,4,4,-Trimethyladipinsäure, Terephthalsäure; Säuren mit ethylenischer Doppelbindung, wie beispielsweise Acrylsäure, Methacrylsäure und Itaconsäure ausgewählt.

Die bei der Acylierung verwendeten Alkansultone sind vorzugsweise Propansulton oder Butansulton, die Salze der Acylierungsmittel sind vorzugsweise die Natriumsalze oder Kaliumsalze.
- Polymeren mit zwitterionischen Einheiten der folgenden Formel: 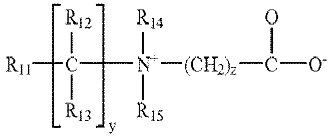 worin R₁₁ eine polymerisierbare ungesättigte Gruppe, wie Acrylat, Methacrylat, Acrylamid oder Methacrylamid, y und z ganze Zahlen von 1 bis 3, R₁₂ und R₁₃ ein Wasserstoffatom, Methyl, Ethyl oder Propyl, R₁₄ und R₁₅ ein Wasserstoffatom oder eine Alkylgruppe bedeuten, die so gewählt ist, dass die Summe der Kohlenstoffatome R₁₄ und R₁₅ 10 nicht übersteigt, sind.

Polymere, die solche Einheiten enthalten, können auch Einheiten aufweisen, die von nicht zwitterionischen Monomeren stammen, wie Dimethyl- und Diethylaminoethylacrylat oder Dimethyl- und Diethylaminoethylmethacrylat oder Alkylacrylate oder Alkylmethacrylate, Acrylamide oder Methacrylamide oder Vinylacetat.
- Polymere, die von Chitosan abgeleitet sind und Monomereinheiten enthalten, die den folgenden Formeln entsprechen: 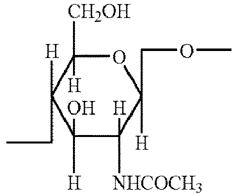 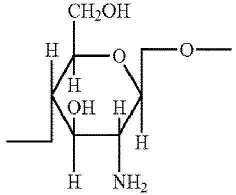 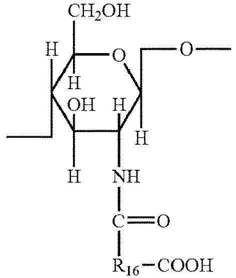 wobei die erste Einheit in Mengenanteilen von 0 bis 30 %, die zweite Einheit in Mengenanteilen von 5 bis 50 % und die dritte Einheit in Mengenanteilen von 30 bis 90 % enthalten ist, mit der Maßgabe, dass in der dritten Einheit R₁₆ eine Gruppe der folgenden Formel bedeutet: 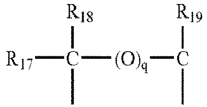 worin falls q = 0, die Gruppen R₁₇, R₁₈ und R₁₉, gleich oder verschieden jeweils ein Wasserstoffatom, Methyl, Hydroxy, Acetoxy oder Amino, einen Monoalkylaminrest oder einen Dialkylaminrest, die gegebenenfalls durch ein oder mehrere Stickstoffatome unterbrochen und / oder gegebenenfalls mit einer oder mehreren Gruppen Amino, Hydroxy, Carboxy, Alkylthio, Sulfonsäure, Alkylthio, dessen Alkylgruppe einen Aminorest trägt, wobei mindestens eine der Gruppen R₁₇, R₁₈ und R₁₉ in diesem Fall ein Wasserstoffatom bedeutet;
   oder falls q = 1, die Gruppen R₁₇, R₁₈ und R₁₉ jeweils ein Wasserstoffatom, sowie die Salze, die diese Verbindungen mit Basen oder Säuren bilden, sind.
- Polymere, die der folgenden allgemeinen Formel entsprechen und die beispielsweise in dem französischen Patent 1 400 366 beschrieben sind: 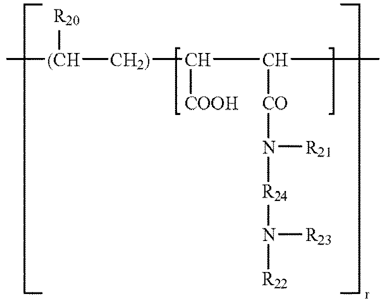 worin R₂₀ ein Wasserstoffatom, CH₃O, CH₃CH₂O oder Phenyl ist, R₂₁ ein Wasserstoffatom oder eine niedere Alkylgruppe, wie Methyl oder Ethyl ist, R₂₂ ein Wasserstoffatom oder eine niedere C₁₋₆-Alkylgruppe, wie Methyl oder Ethyl ist, R₂₃ eine niedere C₁₋₆-Alkylgruppe, wie Methyl oder Ethyl oder eine Gruppe der Formel: -R₂₄-N(R₂₂)₂ ist, wobei R₂₄ eine Gruppe -CH₂-CH₂, -CH₂-CH₂-CH₂- oder -CH₂-CH(CH₃)- darstellt und wobei R₂₂ die oben angegebenen Bedeutungen aufweist.
- Polymere, die bei der N-Carboxyalkylierung von Chitosan gebildet werden können, wie N-Carboxymethylchitosan oder N-Carboxybutylchitosan.
- Alkyl(C₁₋₅)vinylether/Maleinsäureanhydrid-Copolymere, die teilweise durch Semiamidierung mit einem N,N-Dialkylaminoalkylamin wie N,N-Dimethylaminopropylamin oder einem N,N-Dialkylaminoalkohol teilweise modifiziert sind. Diese Polymere können auch weitere Comonomere enthalten, wie Vinylcaprolactam.

Ganz besondere vorteilhafte amphotere Polymere sind z.B. die Copolymere Octylacrylamid / Acrylate / Butylamino-Ethylmethacrylat-Copolymer, die unter den Bezeichnungen AMPHOMER^{®}, AMPHOMER^{®} LV 71 oder BALANCE^{®} 47 der Firma AkzoNobel im Handel sind, und Methylmethacrylat / Methyl-dimethylcarboxymethylammoniumethylmethacrylat-Copolymere.

Es ist gegebenenfalls vorteilhaft, die anionischen und amphoteren Polymere zur Verbesserung ihrer Wasserlöslichkeit bzw. ihrer Wasserdispergierbarkeit mit geeigneten Basen zu neutralisieren.

Als Neutralisationsmittel für Polymere, die Säuregruppen enthalten, können folgende Basen eingesetzt werden: Hydroxide, deren Kation Ammonium oder ein Alkalimetall ist wie z. B. NaOH oder KOH.

Andere Neutralisationsmittel sind primäre, sekundäre oder tertiäre Amine, Aminoalkohole oder Ammoniak. Bevorzugt werden hier 2-Amino-2-methyl-1,3-propandiol (AMPD), 2-Amino-2-ethyl-1,3-propandiol (AEPD), 2-Amino-2-methyl-1-propanol (AMP), 2-Amino-1-butanol (AB), 2-Amino-1,3-propandiol, Monoethanolamin (MEA), Diethanolamin (DEA), Triethanolamin (TEA), Monoisopropanolamin (MIPA), Diisopropanolamin (DIPA), Triisopropanolamin (TIPA), Dimethyl Laurylamin (DML), Dimethyl Myristalamin (DMM), und Dimethyl Stearamin (DMS) verwendet.

Die Neutralisation kann je nach Anwendungszweck teilweise oder vollständig erfolgen.

Bevorzugt einsetzbar sind kationische Polymere, wie beispielweise Polymere, die primäre, sekundäre tertiäre und / oder quaternäre Aminogruppen enthalten, die Teil der Polymerkette oder direkt an die Polymerkette gebunden sind.

Als erfindungsgemäß vorteilhafte kationische Polymere können Polyquaternium-4, Polyquaternium-6, Polyquaternium-7, Polyquaternium-10, Polyquaternium-11, Polyquaternium-16, Polyquaternium-22, Polyquaternium-44, Polyquaternium-46, Polyquaternium-55 und/oder quaternisierte Guarderivate eingesetzt werden.

Erfindungsgemäß besonders bevorzugt ist es, wenn als kationische Polymere Polyquaternium-4, Polyquaternium-16 oder Polyquaternium-44 eingesetzt werden. Beispielsweise können die folgenden kationischen Polyquaternium-Typen eingesetzt werden: Celquat H-100 oder Celquat L-200 (Fa. National Starch); INCI: Polyquaternium-4, Luviquat Excellence, Luviquat FC-370, Luviquat FC-550 oder Luviquat Style (Fa.: BASF); INCI: Polyquaternium-16, Luviquat Care oder Luviquat Ultra Care (Fa. BASF); INCI: Polyquaternium-44.

Weiterhin kann die Haarstyling-Zusammensetzung Haarpflegende Substanzen enthalten. Dabei handelt es sich bevorzugt um kationische Pflegesubstanzen, insbesondere um kationische Tenside.

Der Anteil der Pflegesubstanzen kann ≥ 0 und ≤ 20 Gew-% und insbesondere ≥ 0 und ≤ 10 Gew-%, bezogen auf die gesamte Formulierung, betragen.

Als erfindungsgemäß vorteilhafte kationische Tenside können Verbindungen aus der Gruppe der quaternären Ammoniumverbindungen, insbesondere Benzyltrialkylammoniumsalze oder Alkyltrimethylammoniumsalze, beispielsweise Cetyltrimethylammoniumchlorid oder -bromid oder - methosulfat, Alkyldimethylhydroxyethylammoniumchloride oder -bromide oder - methosulfat, Dialkyldimethylammonium-chloride oder -bromide oder -methosulfat, Esterquats wie z.B. Diacylethyl Hydroxyethylmethylammoniumchlorid oder -bromid oder - methosulfat, Alkylamidethyltrimethylammoniumethersulfate, Hydroxyethyl Cetyldimonium Phosphate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyl-dimethylaminoxide eingesetzt werden.
Erfindungsgemäß besonders bevorzugt ist es, wenn als kationisches Tensid Hydroxyethylcetyldimoniumphosphat eingesetzt wird.
Beispielsweise kann das folgende kationische Hydroxyethylcetyldimoniumphosphat eingesetzt werden: Luviquat Mono CP (Fa. BASF); INCI: Hydroxyethyl Cetyldimonium Phosphate.

Optional können die für Haarstyling-Zusammensetzungen üblichen Additive ebenfalls in der Zusammensetzung enthalten sein. Hierbei kann es sich etwa um Silikone oder Silikonderivate, Benetzungsmittel, Feuchthaltemittel, Weichmacher wie Glycerin, Glykol und Phthalester und -ether, Riechstoffe und Parfüms, UV-Absorber, Farbstoffe, Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, Emulgatoren, oberflächenaktive Mittel, Viskositätsmodifizierer und Rheologiemodifizierer, Geliermittel, Trübungsmittel, Stabilisatoren, Tenside, Sequestierungsmittel, Komplexbildner, Perlglanzmittel, ästhetische Verstärker, Fettsäuren, Fettalkohole, Triglyceride, botanische Extrakte oder Klärhilfsmittel handeln.

Diese Additive können insgesamt in einer Konzentration von etwa ≥ 0 und ≤ 15 Gew.-% vorzugsweise > 0,01 und ≤ 10 Gew.-%, bezogen auf das Gesamtgewicht der Zusammensetzung, vorhanden sein.

Erfindungsgemäß vorteilhafte Ausführungsformen der vorliegenden Erfindung sind auch dadurch gekennzeichnet, dass die Zusammensetzung von 0,01 bis 0,5 Gew.-% Panthenol und/oder Niacinamid enthält, wobei sich die Gewichtsangabe auf das Gesamtgewicht der Zusammensetzung bezieht.

Ein weiterer Gegenstand der Erfindung ist die Verwendung der erfindungsgemäßen Haarstyling-Zusammensetzung zur Formgebung, Festigung und/oder Fixierung der Haare.

Ebenfalls ist ein Verfahren zur Formgebung, Festigung oder Fixierung der Haare unter Verwendung der erfindungsgemäßen Haarstyling-Zusammensetzungen, wobei die Haarstyling-Zusammensetzung auf die Haare aufgetragen wird, Gegenstand der Erfindung.
Vorteilhaft verbleiben bei dem erfindungsgemäßen Verfahren die Haarstyling-Zusammensetzungen zumindest teilweise auf den Haaren.

Die erfindungsgemäß verwendete Dispersion kann selbstverständlich auch für kosmetische Anwendungen wie hautkosmetisch Zusammensetzungen und andere haarkosmetische Anwendungen außer Haarstyling-Produkten eingesetzt werden. Dabei beinhalten die Zusammensetzungen die für die jeweilige Anwendung üblichen Inhaltsstoffe und Additive.

Eine hautkosmetische Zusammensetzung ist definiert als ein kosmetisches Mittel zum Auftragen auf die Haut, beispielsweise zur Reinigung, Verschönerung, Pflege und zum Schutz der Haut. Im Sinne der vorliegenden Erfindung sind hautkosmetische Zusammensetzungen Hautpflegeprodukt, Sonnenschutzmittel, After-sun-Präparate, Selbstbräuner, dekorative Kosmetik, Wasch-, Dusch- und Badepräparate zur Anwendung auf der Haut, Gesichtwasser, Gesichtmasken, Gesichtmasken mit Peel-off, Insect-Repellent-Präparate, Fußpflegemittel, Rasiermittel, Haarentfernungsmittel, Intimpflegemittel, Babypflegemittel, Deodorantien und Antitranspirantien.

Bei einem Hautpflegeprodukt handelt es ich um eine kosmetische Zusammensetzung zum Auftragen auf die Haut, das Gesicht oder/und den Körper zum Schutz gegen Hautveränderungen, beispielweise Hautalterung, Austrocknung usw.

Entsprechend ihrem Aufbau können die erfindungsgemäßen Zusammensetzungen beispielweise als Gesichtscreme, Tages- oder Nachtcreme, Augencreme, Antifaltencreme, Whitening-Produkte, Körperlotion, Tränkungsmedium, After-sun-Präparate usw. verwendet werden. Es ist gegebenenfalls möglich, dass die erfindungsgemäßen Zusammensetzungen als pharmazeutisches Produkt verwendet werden.

Insect-Repellent-Präparat sind im Rahmen der vorliegenden Erfindung Präparate, die zur Abwehr und Vertreibung von Insekten, insbesondere von Mücken, Zecken und Milben, äußerlich verwendet werden. In solchen Formulierungen werden Wirkstoffe eingesetzt, welche die Insekten aufgrund der Bildung eines Duftmantels über der Haut von der Haut entfernt halten.

Bei einem Sonnenschutzmittel handelt es sich um eine Zusammensetzung zum Schutz der Haut gegenüber kurzwelliger und längerwelliger Sonnenstrahlung. Ein Sonnenschutzmittel enthält mindestens eine Lichtschutzfiltersubtanz (UVA-, UVB- und/oder Breitbandfilter). Hautpflegeprodukte enthalten üblicherweise Filmbildner, Feuchthaltmittel und gegebenenfalls weitere kosmetische und/oder dermatologische Wirk-, Hilfs- und Zusatzstoffe, ausgebildet sein.

Insect-Repellent-Präparate, enthalten üblicherweise Filmbildner, Insect-Repellent-Wirkstoffe und gegebenenfalls weitere kosmetische und/oder dermatologische Wirk-, Hilfs- und Zusatzstoffe.

Vorteilhaft werden als Insect-Repellent-Wirkstoffe 3-(N-n-Butyl-N-acetyl-amino)-pro¬pion¬säu¬re¬ethyl-ester (unter der Handelsbezeichnung Repellent 3535 erhältlich), N,N-Ditethyl-m-toluamid (so ge-nannt DEET) und 2-(2-Hydroxyethyl)-piperidin-1-carbonsäure-2-butylester (unter der Handelsbe-zeich¬nung Bayrepel® erhältlich) eingesetzt.

Sonnenschutzmittel enthalten üblicherweise mindestens einen Filmbildner, mindestens eine oder mehrere Lichtschutzfiltersubtanzen und gegebenenfalls weitere kosmetische und/oder dermatologische Wirk-, Hilfs- und Zusatzstoffe.

Lichtschutzfiltersubtanzen könne aus der Gruppe, bestehend aus den UVA-, UVB-, Breitbandfiltern und deren Mischungen, ausgewählt werden.

Selbstbräunungsmittel, enthalten üblicherweise mindestens einen Filmbildner, mindestens ein oder mehrere selbtsbräunende Subtanzen und gegebenenfalls weitere kosmetische und/oder dermatologische Wirk-, Hilfs- und Zusatzstoffe.

Selbstbräunungsmittel enthalten mindestens ein oder mehrere selbtsbräunende Subtanzen, die bevorzugt ausgewählt werden aus der Gruppe, bestehend aus Glycerolaldehyd, Hydroxymethylglyoxal, γ-Dialdehyd, Erythrulose, 5-Hydroxy-1,4-naphtochionon, 2-Hydroxy-1,4-naphtochinon, 1-, 3-Dihydorxyaceton (DHA), 6-Aldo-D-Fructose und Ninhydrin.

Bei einer dekorativen kosmetischen Formulierung handelt es sich um eine kosmetische Zusammensetzung zur farblichen Gestaltung der menschlichen Haut, Schleimhaut, Semi-Schleimhaut, des Haar und des Nagels. Die erfindungsgemäße dekorative Formulierung kann ein Gesichts-Make-up (Foundation), eine getönte (Tages-)Creme, ein Blush, ein Rouge, eine Wimperntusche, ein Eyeliner, ein Kajal, ein Lidschatten, ein Lippenstift, ein Lip-Gloss zur farblichen Veränderung oder zum Schminken des Körpers gegen Augenringe, inhomogenen Teint oder weitere Unvollkommenheiten der Haut wie Rötungen, Flecken, Falten oder Pickel sein. Die Liste von dekorativen Produkten ist selbstverständlich nicht limitierend zu verstehen.

Dekorative kosmetische Zusammensetzungen enthalten üblicherweise mindestens einen Filmbildner, mindestens einem Farbstoff und gegebenenfalls weitere kosmetische und/oder dermatologische Wirk-, Hilfs- und Zusatzstoffe, ausgebildet sein. Die Farbstoffe könne ausgewählt werden aus der Gruppe, bestehend aus löslichen Farbstoffen; anorganischen Pigmenten, wie zum Beispiel Eisenoxiden und Chromoxiden; Ultramarin; Manganviolett; organischen Pigmenten und Perlmutt.

Die erfindungsgemäßen hautkosmetischen Zusammensetzungen können fest (Stick), flüssig (Lotion, Pflegeöl) oder halbfest (Creme, Salben oder gelartige Produkte) sein. Die Zusammensetzungen können beispielweise in Form einer Öl-in-Wasser-, Silikon-in-Wasser- Wasser-in-Öl-, Wasser-in-Silikon-, Öl-in-Wasser-in-Öl- oder Wasser-in-Öl-in-Wasser-Emulsion vorliegen. Die Zusammen-set-zung können ferner mit einem Treibgas aufgeschäumt werden (so genanntes Mousse). Die oben erwähnten Emulsionen können durch O/W-, W/O- oder W/Si-Emulgator, Verdicker (wie beispielweise im Fall einer Hydrodispersion) oder Feststoffe (wie beispielweise Pickeringemulsion) stabilisiert sein. Die erfindungsgemäße Formulierung kann in Form von "loose Pulver" oder Kompaktpulver vorliegen.

Weitere haarkosmetische Anwendungen sind beispielsweise Egalisierungsmittel für Dauerwellen, Curl Relaxer, Haarverformungsmittel, Haarfärbemittel, Haarkuren und Shampoos.

Die vorliegende Erfindung wird an Hand von Beispielen erläutert, wobei sie nicht als einschränkend zu verstehen sind. Alle Mengenangaben, Anteile und Prozentanteile sind, soweit nicht anders angegeben, auf das Gewicht und die Gesamtmenge bzw. auf das Gesamtgewicht der Zusammensetzungen bezogen.

### Beispiele:

Sofern nicht abweichend gekennzeichnet, beziehen sich alle Prozentangaben auf das Gewicht.

Sofern nicht abweichend vermerkt, beziehen alle analytischen Messungen auf Temperaturen von 23°C.
Die Bestimmung der Festkörpergehalte (nicht-flüchtiger Anteil) erfolgte nach DIN-EN ISO 3251.

NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt.

Das zahlenmittlere Molekulargewicht Mₙ wurde bestimmt durch Gelpermeationschromatographie (GPC) in Tetrahydrofuran bei 23°C. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2×PSS SDV linear M, 8×300 mm, 5 µm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Die Glasübergangstemperatur T_{g} wurde mittels dynamischer Differenzkalorimetrie (DSC) in Anlehnung an die DIN EN 61006, Verfahren A, bestimmt, wobei ein DSC Gerät (Kalorimeter Pyris Diamond DSC von Perkin-Elmer) verwendet wird, das zur Bestimmung von T_{g} mit Indium und Blei kalibriert ist. Es werden 10 mg der zu untersuchenden Substanz in einen verschließbaren Aluminium-Tiegel eingewogen und dieser verschlossen. Es werden drei unmittelbar aufeinander folgende Durchläufe einer Aufheizungen von -100°C bis +150°C, Heizrate 20 K/min, mit anschließender Abkühlung Kühlrate 320 K/min vorgenommen und die dritte Aufheizkurve zur Bestimmung der Werte verwendet. Als Tg wird die Temperatur bei der halben Höhe einer Glasübergangsstufe bestimmt.

### Verwendete Substanzen und Abkürzungen:

- Polyesterpolyol 1:: Polyesterpolyol aus Phthalsäureanhydrid und Ethylenglykol, Mₙ = 2000 g/mol, Tg = 21,5 °C, amorphe Struktur
- Polyesterpolyol 2:: Polyesterpolyol aus Adipinsäure und Diethylenglykol, Mₙ = 2700 g/mol, Tg = -52,5°C, amorphe Struktur

Die Polyesterpolyole und IPDI wurden hergestellt von der Bayer MaterialScience AG, Leverkusen, DE. Weitere Chemikalien von Sigma-Aldrich Chemie GmbH, Taufkirchen, DE. Die Rohstoffe wurden, soweit nicht anders erwähnt, ohne weitere Reinigung oder Vorbehandlung eingesetzt.

### Herstellung der Polyurethanharnstoffdispersion:

180,4 g einer Mischung aus Polyesterpolyol 1 und Polyesterpolyol 2 (Komponente b1) und b2) im Gewichtsverhältnis b1)/b2) = 1/2) wurden auf 100°C aufgeheizt und 1 h im Vakuum entwässert. Anschließend wurde abgekühlt und 51,2 g N-Methyldiethanolamin und 13,5 g 1,4-Butandiol zusammen mit 279,2 g Aceton zugegeben. Die Temperatur wurde auf 50 °C eingestellt und 212,3 g IPDI zugegeben und solange bei 50°C gerührt bis der theoretische NCO-Wert leicht unterschritten war. Das fertige Prepolymer wurde mit 41,2 g IPDA, welches gelöst in 150 g Aceton vorlag, bei 40°C umgesetzt. Die Nachrührzeit betrug 15 min. Danach wurde durch Zugabe von 232 g Wasser und 25,8 g 10%iger Essigsäure neutralisiert. Die Rührzeit betrug 30 min bei 50 °C. Anschließend wurde in 668,1 g Wasser bei 25 °C innerhalb von 15 min dispergiert. Es folgte die Entfernung des Lösemittels durch Destillation im Vakuum bei 40 °C und es wurde eine lagerstabile Dispersion erhalten.

| | |
|---|---|
| Feststoffgehalt: | 35% |
| Partikelgröße (LKS): | 314 nm |
| Viskosität: | 89 mPas |

### Anwendungsbeispiel

### Pump-setting-Spray

| | A | B |
|---|---|---|
| Erfindungsgemäßer Po-lyurethanharnstoff (bezogen auf Aktivstoff) | 2 | 10 |
| Ethanol | 55 | 30 |
| Parfüm | q.s. | q.s. |
| Wasser | ad 100 | ad 100 |

### Aerosol-Hair-Sprays

| | C | D | E | F |
|---|---|---|---|---|
| Erfindungsgemäßer Po-lyurethanharnstoff (bezogen auf Aktivstoff) | 5 | 5 | 2 | 8 |
| Panthenol | | | 0,5 | 0,5 |
| PEG-12 dimethicone | | 0,05 | | |
| Cyclomethicone | | | 1,0 | 1,0 |
| Parfüm | q.s | q.s | q.s | q.s |
| Ethanol | ad 100 | 20 | ad 100 | 20 |
| Wasser | | ad 100 | | ad 100 |
| Propan/butan 3,5 Bar. (20°C) | | | | |
| Dimethylether | 40 | 30 | 50 | |
| Fluorkohlenwasserstoff 152 A | | | | 30 |

### Haarschaum

| | G |
|---|---|
| Erfindungsgemäßer Polyurethan-harnstoff (bezogen auf Aktivstoff) | 4 |
| Panthenol | 0,05 |
| Polyquaternium-4 | 2 |
| Cetyltrimethylammoniumchlorid | 0,2 |
| Parfüm | q.s. |
| Ethanol | 6 |
| Wasser | ad 100 |
| Konservierungsmittel | q.s. |
| Propan/Butan 3,5 Bar. (20°C) | 10 |

## Patentansprüche

1. Haarstyling-Zusammensetzung enthaltend wenigstens ein Treibgas und/oder einen Verdicker und eine wässrige Dispersion eines kationisch hydrophilierten Polyurethanharnstoffs, **dadurch gekennzeichnet, dass** der Polyurethanharnstoff aufgebaut ist aus
a) wenigstens einem aliphatischen, araliphatischen und/oder cycloaliphatischen Polyisocyanat,
b) Polyolen mit einem zahlenmittleren Molekulargewicht Mn ≥ 400 und ≤ 6000 g/mol und einer Hydroxylfunktionalität von ≥ 1,5 und ≤ 4, umfassend wenigstens ein Polyesterpolyol b1), das eine amorphe Struktur und eine Glasübergangstemperatur Tg > 10°C, bestimmt mittels dynamischer Differenzkalorimetrie DSC in Anlehnung an die DIN EN 61006, Verfahren A, aufweist,
c) wenigstens einer kationisch hydrophilierenden Komponente, die mindestens eine gegenüber Isocyanatgruppen reaktive Gruppe und mindestens eine kationische oder potentiell kationische Gruppe aufweist,
d) wenigstens einer aliphatischen, aminofunktionellen Verbindung, die mindestens zwei isocyanatreaktive Aminogruppen aufweist,
e) gegebenenfalls wenigstens einem Alkohol, der mindestens zwei Hydroxylgruppen und eine Molmasse ≥ 60 und ≤ 399 g/mol aufweist und
f) gegebenenfalls wenigstens einer Verbindung die eine gegenüber Isocyanatgruppen reaktive Gruppe aufweist.

2. Haarstyling-Zusammensetzung gemäß Anspruch 1, **dadurch gekennzeichnet, dass** der Polyurethanharnstoff einen Gehalt an kationischen und/oder potentiell kationischen Gruppen von ≥ 0,2 und ≤ 5 Milliequivalenten pro g Polymer aufweist.

3. Haarstyling-Zusammensetzung gemäß Anspruch 1 oder 2, **dadurch gekennzeichnet, dass** der Polyurethanharnstoff erhältlich ist durch Umsetzung der Komponenten a), b), c) und gegebenenfalls e) zu einem Isocyanat-terminierten Präpolymer, anschließender Umsetzung des Präpolymers mit der Komponente d) und gegebenenfalls den Komponenten e) und f) und falls die Komponente c) eine potentiell kationische Gruppe umfasst, Neutralisation dieser Gruppe durch eine Säure vor, während oder nach der Umsetzung des Isocyanat-terminierten Präpolymers mit der Komponente d) und gegebenenfalls den Komponenten e) und f).

4. Haarstyling-Zusammensetzung gemäß einem der Ansprüche 1 bis 3, **dadurch gekennzeichnet, dass** die Komponente d) keine ionischen oder potentiell ionischen Gruppen aufweist.

5. Haarstyling-Zusammensetzung gemäß einem der Ansprüche 1 bis 4, **dadurch gekennzeichnet, dass** das Polyesterpolyol b1) erhältlich ist aus Säure- und Alkoholkomponenten, wobei als Säurekomponente ausschließlich aromatische Dicarbonsäuren eingesetzt werden.

6. Haarstyling-Zusammensetzung gemäß einem der Ansprüche 1 bis 5, **dadurch gekennzeichnet, dass** das Polyesterpolyol b1) erhältlich ist aus Phthalsäure und/oder Phthalsäureanhydrid und Ethylenglykol.

7. Haarstyling-Zusammensetzung gemäß einem der Ansprüche 1 bis 6, **dadurch gekennzeichnet, dass** die Komponente b) neben dem Polyesterpolyol b1) ein weiteres Polyesterpolyol b2) umfasst, das bevorzugt als Aufbaukomponente wenigstens eine aliphatische Dicarbonsäure und/oder das entsprechende Carbonsäureanhydrid enthält.

8. Haarstyling-Zusammensetzung gemäß einem der Ansprüche 1 bis 7, **dadurch gekennzeichnet, dass** das Polyesterpolyol b2) eine amorphe Struktur aufweist.

9. Haarstyling-Zusammensetzung gemäß einem der Ansprüche 1 bis 8, **dadurch gekennzeichnet, dass** die Polyesterpolyole b1) und b2) im Verhältnis b1) : b2) von 2:1 bis 1:3 vorliegen.

10. Haarstyling-Zusammensetzung gemäß einem der Ansprüche 1 bis 9, **dadurch gekennzeichnet, dass** diese ≥ 0,2 und ≤ 20 Gew.-% des Polyurethanharnstoffs als Aktivsubstanz, bezogen auf das Gesamtgewicht der Haarstyling-Zusammensetzung, enthält.

11. Haarstyling-Zusammensetzung gemäß einem der Ansprüche 1 bis 10, **dadurch gekennzeichnet, dass** diese als Mousse vorliegt.

12. Verwendung der Haarstyling-Zusammensetzung gemäß einem der Ansprüche 1 bis 11 zur Formgebung, Festigung und/oder Fixierung der Haare.

13. Verfahren zur Formgebung, Festigung oder Fixierung der Haare unter Verwendung von Haarstyling-Zusammensetzungen gemäß einem der Ansprüche 1 bis 11, wobei die Haarstyling-Zusammensetzung auf die Haare aufgetragen wird.

14. Verwendung von wässrigen Dispersion eines kationisch hydrophilierten Polyurethanharnstoffs, der aufgebaut ist aus
a) wenigstens einem aliphatischen, araliphatischen und/oder cycloaliphatischen Polyisocyanat,
b) Polyolen mit einem zahlenmittleren Molekulargewicht Mn ≥ 400 und ≤ 6000 g/mol und einer Hydroxylfunktionalität von ≥ 1,5 und ≤ 4, umfassend wenigstens ein Polyesterpolyol b1), das eine amorphe Struktur und eine Glasübergangstemperatur Tg > 10°C, bestimmt mittels dynamischer Differenzkalorimetrie DSC in Anlehnung an die DIN EN 61006, Verfahren A, aufweist,
c) wenigstens einer kationisch hydrophilierenden Komponente, die mindestens eine gegenüber Isocyanatgruppen reaktive Gruppe und mindestens eine kationische oder potentiell kationische Gruppe aufweist,
d) wenigstens einer aliphatischen, aminofunktionellen Verbindung, die mindestens zwei isocyanatreaktive Aminogruppen aufweist,
e) gegebenenfalls wenigstens einem Alkohol, der mindestens zwei Hydroxylgruppen und eine Molmasse ≥ 60 und ≤ 399 g/mol aufweist,
f) gegebenenfalls wenigstens einer Verbindung die eine gegenüber Isocyanatgruppen reaktive Gruppe aufweist
in Haarstyling-Zusammensetzungen.
